# FASCICULE DE BREVET EUROPEEN

(11) **EP 1 642 541 B1**
(45) Date de publication et mention de la délivrance du brevet: **27.06.2007**
(21) Numéro de dépôt: 05356168.4
(22) Date de dépôt: 23.09.2005
(51) Int. Cl.: A61B 17/32

(54) **Dispositif de prélèvement et d'implantation de greffons capillaires**
Vorrichtung zur Entnahme und Implantation von Haartransplantat
Device for cutting and implanting hair grafts

(30) Priorité: 04.10.2004 FR 0410449
(43) Date de publication de la demande: 05.04.2006
(73) Titulaire: Pitot, Patrick Eric, 63550 Saint Victor Montvianeix (FR)
(72) Inventeur: Pitot, Patrick Eric, 63550 Saint Victor Montvianeix (FR)
(74) Mandataire: Myon, Gérard Jean-Pierre

(56) Documents cités:
- EP-A- 1 312 315
- FR-A- 2 776 180
- US-A- 5 129 913
- US-A- 5 458 608

## Description

L'invention a trait à un dispositif de prélèvement et d'implantation de greffons capillaires.

On connaît de tels dispositifs utilisés pour prélever, sur la tête d'un individu, un ou plusieurs cheveux, avec le bulbe capillaire correspondant et une partie du cuir chevelu. Ces dispositifs permettent le prélèvement, la manipulation et l'implantation du greffon dans une zone du crâne dépourvue de cheveux. De tels dispositifs sont utilisés lors d'une intervention chirurgicale visant à remédier aux calvities, notamment les calvities partielles.

On connaît des dispositifs, tels ceux décrits dans FR-A-2 816 823, où un organe mobile de prélèvement se déplace sous l'action d'un fluide sous pression. Le retour de l'organe à sa position initiale se fait sous l'action d'un ressort. On connaît également des dispositifs, tels ceux décrits dans EP-A-1 312 315, où le déplacement de l'organe de prélèvement est effectué manuellement, le rappel en position se faisant grâce à des ressorts.

D'autres dispositifs, tels ceux décrits dans FR-A-2 776 180, sont adaptés pour un prélèvement du greffon par aspiration à l'aide d'une source de vide. Un ressort à compression permet également le rappel en position de l'organe de prélèvement formant un piston coulissant dans le corps de l'appareil.

Ces différents dispositifs, notamment ceux faisant appel à un fluide sous pression ou travaillant par aspiration, nécessitent un appareillage relativement encombrant, dont les conduits ne permettent pas une utilisation aisée par le praticien. De plus, la présence de ressorts de rappel en position de l'organe de prélèvement du greffon, ne permet pas une continuité et une douceur du geste chirurgical. En effet, il arrive que lors du rappel celui-ci soit mal contrôlé, ce qui peut entraîner une détérioration ou une perte du greffon. Par ailleurs, ces dispositifs ne sont pas aisément stérilisables.

C'est à ces inconvénients qu'entend plus particulièrement remédier l'invention en proposant un dispositif de prélèvement et d'implantation de greffons capillaires, d'une manipulation aisée, peu encombrant et dont le retour en position de l'organe de prélèvement est continu et peut être contrôlé à tout moment par le praticien.

A cet effet, l'invention a pour objet un dispositif de prélèvement et d'implantation de greffons capillaires comprenant un corps creux formant un logement adapté pour recevoir une aiguille creuse, le dispositif étant équipé d'un moyen de manoeuvre permettant de déplacer l'aiguille entre une première position, dite active, de prélèvement de greffons et une seconde position, dite de repos, d'implantation de greffons, caractérisé en ce qu'il est pourvu de moyens de guidage de l'aiguille selon un mouvement sensiblement hélicoïdal entre les première et seconde positions.

Grâce à l'invention, on réalise un dispositif où le passage, entre la position de repos de l'aiguille et la position active de l'aiguille s'effectue de manière continue, régulière et contrôlée.

Selon des aspects avantageux, mais non obligatoires, de l'invention un tel dispositif de prélèvement et d'implantation peut incorporer une ou plusieurs des caractéristiques suivantes :
- Le corps formant un logement est un cylindre creux à section circulaire dont l'axe principal est globalement confondu avec un axe longitudinal de l'aiguille.
- Le mouvement sensiblement hélicoïdal de l'aiguille s'effectue autour d'un axe formé d'une tige, fixée à une extrémité du corps et disposée coaxialement dans l'aiguille, la tige dépassant du débouché de l'aiguille lorsque celle-ci est en position de repos.
- Les moyens de guidage comprennent une fente ménagée dans la paroi du corps, cette fente étant inclinée par rapport à l'axe longitudinal du corps.
- Les moyens de guidage comprennent une fente, en arc de cercle, ménagée à une extrémité du corps.
- Les moyens de guidage comprennent un organe de manoeuvre, notamment un bouton ou un pion, apte à coulisser à jeu réduit dans la fente.
- La fente est pourvue, à au moins une de ses extrémités, d'une zone de réception et de maintien du pion.
- Les moyens de guidage comprennent au moins une rainure hélicoïdale ménagée sur au moins une partie de la paroi interne du corps.
- Les moyens de guidage comprennent des reliefs hélicoïdaux sur au moins une partie de la paroi externe de l'aiguille et adaptés pour coopérer avec les rainures.
- L'extrémité de l'aiguille est biseautée.

L'invention sera mieux comprise et d'autres avantages de celle-ci apparaîtront plus clairement, à la lumière de la description qui va suivre de trois modes de réalisation d'un dispositif d'implantation et de prélèvement d'un greffon capillaire conforme à son principe, donnée uniquement à titre d'exemple et faite en référence aux dessins annexés dans lesquels :
- la figure 1 est une vue en perspective d'un dispositif conforme à un premier mode de réalisation de l'invention, l'aiguille étant dans une position intermédiaire entre une position active et une position de repos,
- la figure 2 est une coupe longitudinale selon le plan Il,
- la figure 3 est une vue similaire à la figure 1, l'aiguille étant en position dite de repos, le dispositif étant représenté en situation au dessus d'une partie du cuir chevelu,
- la figure 4 est une vue analogue à la figure 3, l'aiguille étant en position dite active, une partie du cuir chevelu étant représentée en arraché,
- la figure 5 est une coupe longitudinale, similaire à la figure 2, d'un dispositif conforme à un deuxième mode de réalisation de l'invention,
- la figure 6 est une coupe longitudinale, similaire à la figure 2, d'un dispositif conforme à un troisième mode de réalisation de l'invention et
- la figure 7 est une vue en perspective partielle du dispositif représenté à la figure 6.

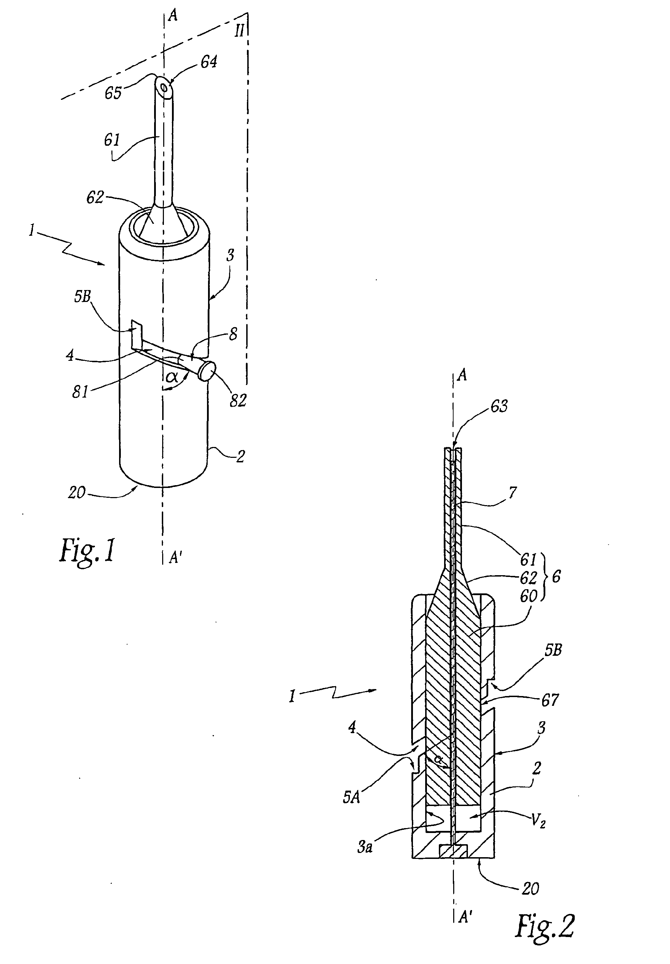

Le dispositif 1 représenté à la figure 1 comprend un corps formé par un cylindre 2 creux, à base circulaire et fermé par un fond 20. Ce cylindre 2 est équipé sur une partie de sa paroi 3 d'une fente 4 traversante. Cette fente 4 traversante est disposée selon une direction inclinée par rapport à un axe longitudinal A-A' du cylindre. L'angle d'inclinaison α de la fente 4 est compris entre 10 degrés et 80 degrés. Cette fente 4 est également équipée, à chacune de ses extrémités d'une encoche 5A et 5B. Ainsi, La fente 4 est globalement configurée en S ou en tronçon d'hélice avec deux encoches d'extrémité.

Une aiguille 6 est disposée à l'intérieur du cylindre 2. Cette aiguille 6 a une forme globalement cylindrique à section circulaire. Elle est formée de deux sections tubulaires 60 et 61, de diamètres extérieurs différents, reliées par une partie tronconique 62. Le diamètre externe de la section 60, de plus fort diamètre, est légèrement inférieur au diamètre interne du corps 2.

Ainsi, l'aiguille 6 s'insère avec un minimum de jeu dans le corps 2 tout en étant montée coulissante dans ce dernier. Dans cette configuration, son axe longitudinal est globalement confondu avec l'axe A-A' du cylindre 2.

L'extrémité 64 de la section 61 située à l'opposé de la section tronconique 62 est ouverte et biseautée.

Comme il apparaît aux figures 2 à 4, l'aiguille 6 est pourvue d'un canal central 63 adapté pour recevoir à jeu réduit une tige 7 fixée au fond 20 du corps 2. La tige 7 est à section circulaire, comme le canal 63, et son axe longitudinal est confondu avec l'axe A-A'.

L'aiguille 6, la tige 7 et le corps cylindrique 2 sont réalisés en un matériau rigide, aisé à nettoyer, à stériliser, et présentant une compatibilité médicale et alimentaire. Avantageusement, l'aiguille 6 et la tige 7 sont réalisées en acier ou en un métal ou un alliage de métaux inoxydables. Le cylindre 2 peut être réalisé dans le même matériau ou dans un autre matériau, par exemple, un polymère ou un composé à base de polymères.

La paroi externe de la section 60 de l'aiguille est équipée d'un organe de manoeuvre 8, en forme de bouton ou de pion, adapté pour coulisser, avec un minimum de jeu, dans la fente 4. Avantageusement, le pion 8 a une forme cylindrique. Une extrémité 81 du pion 8 est fixée sur la paroi de la section 60 et son autre extrémité 82 fait saillie radialement, par la fente 4, à l'extérieur du corps 2. Cette extrémité 82 est aplatie en forme de disque afin de faciliter la manoeuvre du pion 8. Dans sa partie courante, le pion 8 a un diamètre légèrement inférieur à la largeur de la fente 4. Ainsi, le pion 8 peut aisément être déplacé d'une extrémité à l'autre de la fente 4 et maintenu en position en bout de fente par insertion dans une des encoches 5A ou 5B.

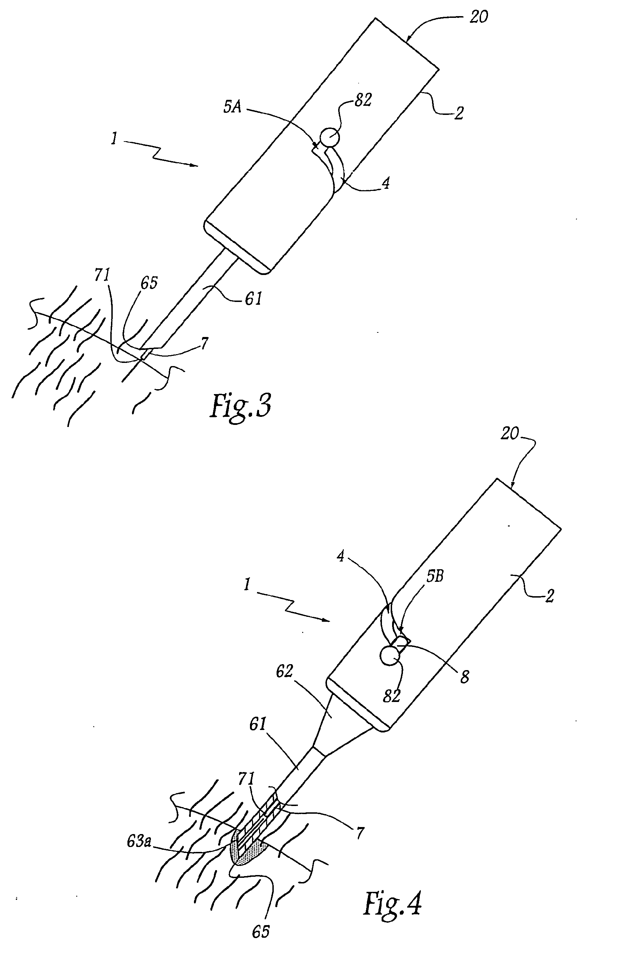

Comme représenté à la figure 3, lorsque l'aiguille est en position de repos, le pion 8 est inséré dans l'encoche 5A de l'extrémité de la fente 4 située le plus près du fond 20 du corps 2. Dans cette position, l'aiguille 6 se trouve rétractée au maximum dans son logement formé par le volume intérieur V₂ du corps 2. La section tronconique 62 de l'aiguille est positionnée dans le corps 2. La tige 7 traverse de part en part le canal 63 et elle dépasse de l'extrémité 64 biseautée de la section 61 de l'aiguille 6.

En position dite active, illustrée à la figure 4, le pion 8 se trouve à l'extrémité de la fente 4 située le plus loin du fond 20 du corps 2. Le pion 8 est maintenu dans cette position par insertion dans l'encoche 5B. Dans cette position, La section 62 de l'aiguille 6 est en partie sortie du corps 2. La tige 7 n'occupe qu'une partie du canal 63 de l'aiguille 6 et elle ne dépasse pas de l'extrémité 64 de la section 61.

Lorsqu'un praticien désire prélever un greffon capillaire sur le crâne d'un patient, il prend, d'une seule main, le dispositif 1 en le positionnant dans la position de repos, représentée figure 3, où l'aiguille 6 est en place dans le corps 2. Dans cette position, l'extrémité 71 de la tige 7 qui dépasse de l'extrémité 64 permet de positionner précisément le dispositif 1 au dessus du greffon à prélever. L'extrémité avant 71 de la tige 7 est ainsi utilisée comme un « pointeur », ce qui permet d'insérer le greffon dans l'extrémité 63a du canal 63 ménagée dans l'extrémité 64.

Le praticien déplace ensuite le pion 8 dans la fente 4 de manière à l'amener vers l'autre extrémité de cette dernière, dans une position où l'extrémité 64 de l'aiguille 6 dépasse de l'extrémité 71 de la tige. Ce mouvement est contrôlé en permanence par le praticien, grâce au pion 8. Lors de ce mouvement, l'aiguille 6 effectue un déplacement hélicoïdal d'axe A-A'. Ainsi, l'extrémité biseautée 64 de l'aiguille 6, en appui sur le cuir chevelu, pénètre dans ce dernier par un mouvement s'apparentant à un vissage. Le bord 65 de l'extrémité biseautée 64 de l'aiguille 6 étant suffisamment fin et tranchant, celle-ci se comporte comme un emporte pièce.

A la fin du mouvement, c'est-à-dire lorsque le pion 8 se trouve bloqué dans l'encoche 5B de l'autre extrémité de la fente 4, comme illustré à la figure 4, l'extrémité biseautée 64 a pénétré suffisamment profondément dans le cuir chevelu et le greffon se trouve, pour sa plus grande partie, logé dans l'extrémité 63a du canal 63.

En maintenant le pion 8 dans cette position, maintien qui est facilité par la présence de l'encoche 5B, le praticien dégage l'ensemble du dispositif du cuir chevelu, ce qui a pour effet de prélever le greffon avec son bulbe capillaire. Il suffit alors au praticien d'amener le dispositif avec le greffon au dessus de l'endroit choisi pour effectuer l'implantation. Une fois en position, le praticien enfonce l'extrémité 64 de l'aiguille dans le cuir chevelu. Par un mouvement hélicoïdal inverse de l'aiguille 6, obtenu en ramenant le pion 8 vers l'encoche 5A de la fente 4, on ramène l'aiguille 6 à sa position dite de repos. Ainsi, le greffon est poussé vers l'extrémité 64 de l'aiguille 6 par la tige 7 qui reste fixe par rapport au corps 2, alors que l'aiguille se déplace en direction du fond 20. A la fin du mouvement, le greffon est dégagé de l'aiguille 6 et il se trouve en place dans le cuir chevelu du patient.

Lors de cette opération, le déplacement de l'aiguille et la mise en place sont contrôlés, en permanence, par le praticien grâce à son action sur le pion 8. Ainsi, le praticien dispose d'un outil simple, aisé à manipuler, de faible encombrement et qui permet un contrôle continu et progressif des contraintes appliquées au greffon, cela à tout moment de l'opération. Il est à noter que, dans une configuration courante, le diamètre intérieur de l'aiguille 6 est d'environ 1,5 mm et que la course totale, entre les deux positions, de l'extrémité 64 de l'aiguille 6 est d'environ 20 mm.

En variante, une encoche 5A ou 5B peut être prévue à une seule extrémité de la fente 4.

Le nombre restreint de pièces constituant le dispositif 1, la nature des matériaux utilisés et l'absence de pièces sensibles à la chaleur permet une stérilisation aisée du dispositif.

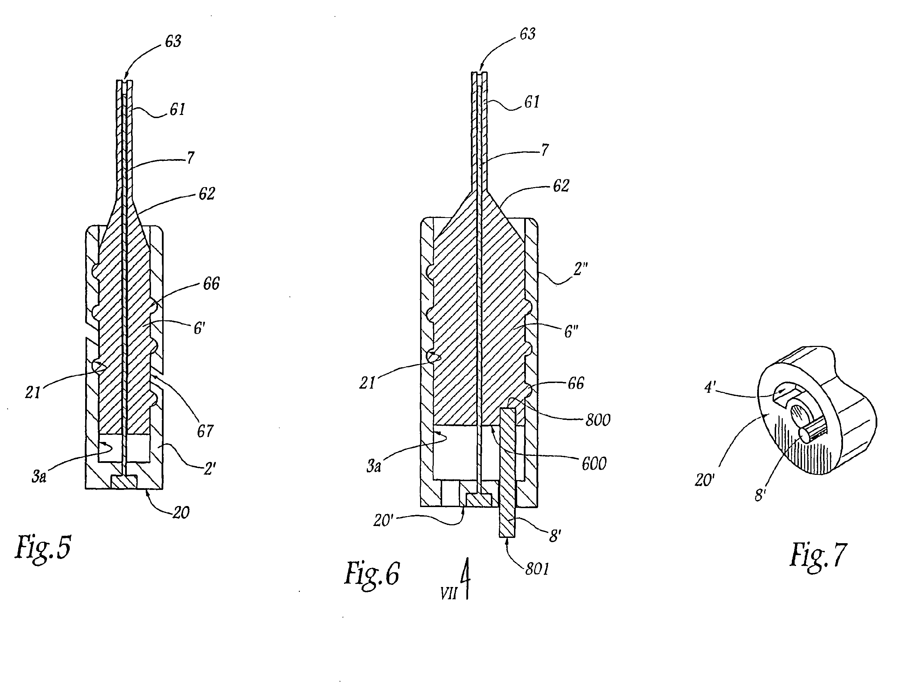

Dans le deuxième mode de réalisation représenté à la figure 5, le mouvement de l'aiguille 6' est guidé et facilité par la présence d'un taraudage hélicoïdal 21 ménagé sur la face interne 3a de la paroi 3 du corps 2'. Ce taraudage 21 est apte à recevoir un filet 66 ménagé sur la paroi extérieure 67 de l'aiguille 6'. Ce mode de réalisation permet une progression et un contrôle encore plus sensibles et précis du mouvement lors de l'utilisation de l'appareil.

En effet, lors de l'utilisation, le praticien a en permanence un doigt sur le pion 8 lors des mouvements de l'aiguille, ce qui permet un contrôle permanent du mouvement.

La présence du taraudage 21 et du filet 66 permet un guidage précis, sans à coup et régulier du déplacement du pion 8 dans la fente 4.

Dans le troisième mode de réalisation représenté aux figures 6 et 7, un pion de manoeuvre 8' se déplace dans une fente 4', ménagée dans le fond 20' du corps 2". Cette fente 4' est disposée en arc en cercle dans le fond 20', au voisinage de sa périphérie. Le pion 8' est solidaire, par son extrémité 800, du fond 600 de l'aguille 6". L'extrémité 801 opposée, s'étend à travers la fente 4' hors du fond 20'. Le pion 8' a une forme similaire au pion 8 et une longueur suffisante pour être manipulé par le praticien. Comme précédemment, le cylindre 2" est pourvu d'un taraudage interne 21 coopérant avec un filet 66 situé sur la paroi externe de l'aiguille 6". Avec un tel dispositif la manoeuvre se fait par déplacement du pion 8' dans la fente 4', ce qui entraîne un mouvement hélicoïdal de l'aiguille 6'.

Selon une variante non représentée, l'extrémité fermée 20 ou 20' du corps 2 ; 2' ; 2" peut être obturée de manière réversible, de façon à permettre, par exemple par dévissage, l'accès à l'intérieur du corps 2 ; 2' ; 2" pour nettoyer ou changer les pièces.

Si le pion 8 ou 8' est monté de manière amovible sur la paroi extérieure de l'aiguille 6 ; 6' ou 6" il est possible de démonter complément le dispositif 1, ce qui permet, par exemple, de changer l'aiguille. Ainsi, on peut utiliser une aiguille 6 ; 6' ou 6" dont le diamètre intérieur est supérieur ou inférieur à celui décrit et/ou dont la longueur est différente. On peut également changer la tige 7. Ainsi, un même dispositif 1 est totalement adaptable aux dimensions du greffon.

La forme extérieure et les dimensions de l'aiguille et du corps cylindrique peuvent être différentes de celles décrites.

## Revendications

1. Dispositif de prélèvement et d'implantation de greffons capillaires comprenant un corps creux (2 ; 2'; 2") formant un logement adapté pour recevoir une aiguille creuse (6 ; 6' ; 6"), ledit dispositif étant équipé d'un moyen de manoeuvre permettant de déplacer ladite aiguille (6 ; 6'; 6") entre une première position, dite active, de prélèvement de greffons et une seconde position, dite de repos, d'implantation de greffons, **caractérisé en ce qu**'il est pourvu de moyens de guidage (4 ; 4' ; 8 ; 8' ; 21 ; 66) de ladite aiguille (6 ; 6' ; 6") selon un mouvement sensiblement hélicoïdal entre lesdites première et seconde positions.

2. Dispositif selon la revendication 1, **caractérisé en ce que** ledit corps formant un logement est un cylindre (2 ; 2'; 2") creux à section circulaire dont l'axe principal (A-A') est globalement confondu avec un axe longitudinal de l'aiguille (6 ; 6'; 6").

3. Dispositif selon la revendication 1, **caractérisé en ce que** le mouvement sensiblement hélicoïdal de ladite aiguille (6 ; 6' ; 6") s'effectue autour d'un axe formé d'une tige (7), fixée à une extrémité (20 ; 20') dudit corps (2 ; 2'; 2") et disposée coxialement dans l'aiguille (6 ; 6' ; 6"), ladite tige (7) dépassant (71) du débouché (64) de l'aiguille (6 ; 6' ; 6") lorsque celle-ci est en position de repos.

4. Dispositif selon l'une des revendications 1 à 3, **caractérisé en ce que** lesdits moyens de guidage (4, 8) comprennent une fente (4) ménagée dans la paroi (3) dudit corps (2 ; 2'), ladite fente étant inclinée (α) par rapport à l'axe longitudinal (A-A') du corps.

5. Dispositif selon l'une des revendications 1 à 3, **caractérisé en ce que** les moyens de guidage (4', 8') comprennent une fente (4'), en arc de cercle, ménagée à une extrémité (20') du corps (2").

6. Dispositif selon l'une des revendications 4 ou 5, **caractérisé en ce que** lesdits moyens de guidage (4, 8; 4', 8') comprennent un organe de manoeuvre, notamment un bouton ou un pion (8; 8') apte à coulisser à jeu réduit dans ladite fente (4 ; 4').

7. Dispositif selon la revendication 6, **caractérisé en ce que** ladite fente (4) est pourvue, à au moins une de ses extrémités, d'une zone (5A, 5B) de réception et de maintien dudit pion (8).

8. Dispositif selon la revendication 1, **caractérisé en ce que** les moyens de guidage comprennent au moins une rainure hélicoïdale ménagée (21) sur au moins une partie de la paroi interne (3a) du corps (2' ; 2").

9. Dispositif selon la revendication 8, **caractérisé en ce que** les moyens de guidage comprennent des reliefs hélicoïdaux (66) sur au moins une partie de la paroi externe (67) de l'aiguille (6 ; 6' ; 6") et adaptés pour coopérer avec lesdites rainures.

10. Dispositif selon l'une des revendications précédentes, **caractérisé en ce que** l'extrémité (64) de l'aiguille (6 ; 6' ; 6") est biseautée.

## Claims

1. Device for taking and implanting hair grafts, comprising a hollow body (2; 2'; 2") forming a housing adapted to receive a hollow needle (6; 6'; 6"), the said device being equipped with a control means permitting displacement of the said needle (6; 6'; 6") between a first position, called the active position, for taking grafts and a second position, called the rest position, for implanting grafts, **characterised in that** it is provided with guide means (4; 4'; 8; 8'; 21; 66) for guiding the said needle (6; 6'; 6") in a substantially helical movement between the said first and second positions.

2. Device according to Claim 1, **characterised in that** the said body forming a housing is a circular-section hollow cylinder (2; 2'; 2"), the principal axis (A-A') of which is generally coincident with a longitudinal axis of the needle (6; 6' ; 6").

3. Device according to Claim 1, **characterised in that** the substantially helical movement of the said needle (6; 6'; 6") takes place about an axis formed by a rod (7), fixed to one end (20; 20') of the said body (2; 2'; 2") and arranged coaxially in the needle (6; 6'; 6"), the said rod (7) projecting (71) from the mouth (64) of the needle (6; 6'; 6") when the latter is in the rest position.

4. Device according to one of Claims 1 to 3, **characterised in that** the said guide means (4, 8) comprise a slot (4) formed in the wall (3) of the said body (2; 2'), the said slot being inclined (α) with respect to the longitudinal axis (A-A') of the body.

5. Device according to one of Claims 1 to 3, **characterised in that** the guide means (4', 8') comprise a circular-arc-shaped slot (4') formed at one end (20') of the body (2") .

6. Device according to one of Claims 4 and 5, **characterised in that** the said guide means (4; 8; 4'; 8') comprise a control member, in particular a button or a pin (8; 8') capable of sliding with limited play in the said slot (4; 4').

7. Device according to Claim 6, **characterised in that** the said slot (4) is provided, at at least one of its ends, with a zone (5A, 5B) for receiving and retaining the said pin (8).

8. Device according to Claim 1, **characterised in that** the guide means comprise at least one helical groove formed (21) on at least part of the inner wall (3a) of the body (2'; 2") .

9. Device according to Claim 8, **characterised in that** the guide means comprise helical protrusions (66) on at least part of the outer wall (67) of the needle (6; 6'; 6") and adapted to cooperate with the said grooves.

10. Device according to one of the preceding claims, **characterised in that** the end (64) of the needle (6; 6' ; 6") is bevelled.

## Patentansprüche

1. Vorrichtung zur Entnahme und zur Implantation von Haartransplantaten, welche einen hohlen Körper (2; 2'; 2") umfasst, der einen Sitz bildet, welcher für die Aufnahme einer hohlen Kanüle (6; 6'; 6") ausgelegt ist, wobei die genannte Vorrichtung mit einem Betätigungsmittel ausgestattet ist, welches das Bewegen der genannten Kanüle (6; 6'; 6") zwischen einer ersten Stellung, der sogenannten aktiven Stellung, für die Entnahme von Transplantaten, und einer zweiten Stellung, der sogenannten Ruhestellung, für die Implantation von Transplantaten ermöglicht, **dadurch gekennzeichnet, dass** Führungsmittel (4; 4; 8; 8', 21; 66) der genannten Kanüle (6; 6'; 6") zwecks einer deutlich schraubenförmigen Bewegung zwischen der genannten ersten und zweiten Stellung vorhanden sind.

2. Vorrichtung gemäß Anspruch 1, **dadurch gekennzeichnet, dass** der genannte, einen Sitz bildende Körper ein Hohlzylinder (2; 2'; 2") mit kreisförmigem Querschnitt ist, dessen Hauptachse (A-A') mit einer Längsachse der Kanüle (6; 6'; 6") gänzlich zusammenfällt.

3. Vorrichtung gemäß Anspruch 1, **dadurch gekennzeichnet, dass** die deutlich schraubenförmige Bewegung der genannten Kanüle (6; 6'; 6") um eine Achse erfolgt, die durch einen Stift (7) gebildet wird, welcher an einem Ende (20; 20') des genannten Körpers (2; 2'; 2") befestigt ist und koaxial in der Kanüle (6; 6'; 6") angeordnet ist, wobei der genannte Stift (7) über die Austrittsstelle (64) der Kanüle (6; 6'; 6") hinausragt (71), wenn sich diese in Ruhestellung befindet.

4. Vorrichtung gemäß einem der Ansprüche 1 bis 3, **dadurch gekennzeichnet, dass** die genannten Führungsmittel (4; 8) einen Spalt (4) umfassen, der in der Wandung (3) des genannten Körpers (2; 2') angelegt ist, wobei der genannte Spalt zur Längsachse (A-A') des Körpers um einen Winkel (α) geneigt ist.

5. Vorrichtung gemäß einem der Ansprüche 1 bis 3, **dadurch gekennzeichnet, dass** die Führungsmittel (4'; 8') einen Spalt (4') in Kreisbogenform umfassen, der an einem Ende (20') des Körpers (2") angelegt ist.

6. Vorrichtung gemäß einem der Ansprüche 4 oder 5, **dadurch gekennzeichnet, dass** die genannten Führungsmittel (4; 8; 4'; 8') eine Betätigungsvorrichtung umfassen, insbesondere einen Betätigungsknopf oder - stift (8; 8'), die imstande ist, mit vermindertem Spiel in dem genannten Spalt (4; 4') zu gleiten.

7. Vorrichtung gemäß Anspruch 6, **dadurch gekennzeichnet, dass** der genannte Spalt (4) an mindestens einem seiner Enden einen Bereich (5A; 5B) zur Aufnahme und zum Verbleib des genannten Stifts (8) aufweist.

8. Vorrichtung gemäß Anspruch 1, **dadurch gekennzeichnet, dass** die Führungsmittel mindestens eine schraubenförmige Rille (21) umfassen, welche auf mindestens einem Teil der Innenwand (3a) des Körpers (2'; 2") angelegt ist.

9. Vorrichtung gemäß Anspruch 8, **dadurch gekennzeichnet, dass** die Führungsmittel auf mindestens einem Teil der Außenwandung (67) der Kanüle (6; 6'; 6") schraubenförmige Reliefs aufweisen und so ausgelegt sind, dass sie mit den genannten Rillen zusammenwirken.

10. Vorrichtung gemäß einem der vorangehenden Ansprüche , **dadurch gekennzeichnet, dass** das Ende (64) der Kanüle (6; 6'; 6") abgeschrägt ist.
